# FASCICULE DE BREVET EUROPEEN

(11) **EP 3 110 957 B1**
(45) Date de publication et mention de la délivrance du brevet: **24.04.2019**
(21) Numéro de dépôt: 15713196.2
(22) Date de dépôt: 26.02.2015
(51) Int. Cl.: C12P 7/64, C12P 17/06

(54) **PROCÉDÉ DE PRODUCTION DE LACTONES À PARTIR D'UNE SOUCHE D'AUREOBASIDIUM PULLULANS**
VERFAHREN ZUR HERSTELLUNG VON LACTONEN AUS EINEM AUREOBASIDIUM-PULLULANS-STAMM
METHOD FOR PRODUCING LACTONES FROM A STRAIN OF AUREOBASIDIUM PULLULANS

(30) Priorité: 27.02.2014 FR 1400499
(43) Date de publication de la demande: 04.01.2017
(73) Titulaire: Charabot, 06131 Grasse (FR)
(72) Inventeur: ALPHAND, Véronique, F-13010 Marseille (FR); ARCHELAS, Alain, F-13001 Marseille (FR); BOUKHRIS-UZAN, Eva, F-13008 Marseille (FR); COURVOISIER-DEZORD, Elise, F-13170 Les Pennes Mirabeau (FR); LAVOINE-HANNEGUELLE, Sophie, F-06370 Mouans Sartoux (FR)
(74) Mandataire: Macquet, Christophe
(86) Numéro de dépôt international: PCT/FR2015/000042
(87) Numéro de publication internationale: WO 2015/128552

(56) Documents cités:
- TAKAFUMI KUROSAWA ET AL: "Extracellular Accumulation of the Polyol Lipids, 3,5-Dihydroxydecanoyl and 5-Hydroxy-2-decenoyl Esters of Arabitol and Mannitol, by Aureobasidium sp.", BIOSCIENCE, BIOTECHNOLOGY, AND BIOCHEMISTRY, vol. 58, no. 11, 12 janvier 1994 (1994-01-12), pages 2057-2060, XP055149907, ISSN: 0916-8451, DOI: 10.1271/bbb.58.2057 cité dans la demande
- ABELOVSKA LENKA ET AL: "Comparison of element levels in minimal and complex yeast media", CANADIAN JOURNAL OF MICROBIOLOGY, NRC RESEARCH PRESS, CA, vol. 53, no. 4, 1 avril 2007 (2007-04-01), pages 533-535, XP008173040, ISSN: 0008-4166, DOI: 10.1139/W07-012

## Description

L'invention concerne un procédé de production de lactones à partir d'une souche d'*Aureobasidium pullulans,* et plus particulièrement de (R)-5,6-dihydro-6-pentyl-2H-pyran-2-one, communément connue sous le nom de (R)-(-)-massoia lactone.

De façon générale, une lactone est un hétérocycle oxygéné, provenant de la cyclisation (ou lactonisation) d'hydroxy-acides. Les principales lactones comportent des cycles entre 4 et 12 atomes de carbone. Leur diversité repose sur la chiralité de ces molécules, la nature des groupements latéraux, ainsi que sur la présence ou non d'insaturation au niveau de la chaîne latérale ou du cycle (Dufossé et al., Importance des lactones dans les arômes alimentaires : structure, distribution, propriétés sensorielles et biosynthèse, 1994).

Les lactones sont des composés aromatiques présents dans une grande variété de produits alimentaires et de boissons.

On s'intéresse plus particulièrement à la massoia lactone. Elle est présente sous forme d'un mélange d'énantiomères en proportions variables. Pour éviter tout doute, en l'absence de précision, les deux formes sont censées être couvertes par le terme massoia lactone tel qu'utilisé dans la présente demande.

On s'intéresse encore plus particulièrement à la (R)-(-)-massoia lactone, également connue sous le nom de (R)-5,6-dihydro-6-pentyl-2H-pyran-2-one ou cocolactone, de structure chimique suivante :

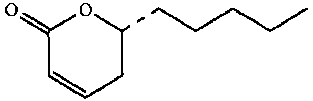

La massoia lactone est une alkyle lactone obtenue à partir de l'huile d'écorce de l'arbre *Cryptocaria massoia* (Abe et al., Studies on the Essential Oil of Masooi, 1937 ; ou encore Rali et al., Comparative chemical analysis of the essential oil constituents in the bark, heartwood and fruits of Cryptocarya massoy (Oken) Kosterm. (*Lauraceae*) from Papua New Guinea, 2007) que l'on trouve notamment en Indonésie.

Par exemple, on la trouve disponible commercialement chez Charabot en tant qu'extrait de l'écorce de l'arbre *Cryptocaria massoia.*

Ce composé peut également être trouvé en tant que composant de la mélasse de canne à sucre (Hashizume et al., Constituents of Cane Molasses, 1968), du tabac séché et de l'huile essentielle d'*Osmanthus fragrans.*

La massoia lactone est principalement connue pour son odeur décrite comme présentant la senteur de la noix de coco, sucrée, crémeuse, laiteuse et cireuse, et son goût décrit comme un arôme noix de coco, crémeux et légèrement fruité. A cet effet, elle est largement utilisée de par ses caractéristiques intrinsèques dans un large éventail de domaines tels que par exemple les produits alimentaires.

Elle est également connue pour son utilisation comme antifongique (document brevet US6060507) ou encore pour son rôle pour attirer les insectes et ainsi faciliter la dispersion des spores (Nago et al., An ecological role of volatiles produced by Lasiodiplodia theobromae, 1994).

La massoia lactone est communément fabriquée par l'extraction de *Cryptocaria massoia* ou par synthèse.

Toutefois, le procédé de fabrication actuel d'origine végétale entraine souvent l'abattage d'un grand nombre d'arbres, et n'est pas adapté à une vaste offre. De plus, un tel procédé est soumis à des contraintes environnementales du fait que l'arbre est souvent tué pendant le processus d'écorçage et entraine des coûts de production très élevés à partir des produits naturels.

La massoia lactone peut ainsi être alternativement synthétisée chimiquement à la fois sous forme de racémate (par exemple Garnero et al., Flavouring substances : design of 6-alkyl-(and 6,6-dialkyl-)5,6-dihydro-2-pyrones, 1986 ; ou encore JP 63-57583, JP 63-215676, JP 63-222164) ou d'énantiomère (par exemple Yu et al., Enantioselective total synthesis of 6R-(-)massoialactone, 1993 ; Takano et al., An enantiospecific route to (6R)-(-)-massoialactone and (4R,6R)-(+)-4-hydroxy-6-pentylvalerolactone, 1992 ; Bennett et al., Total syntheses of natural (+)-(4R,6R)-4-hydroxy-6-pentylvalerolactone and of (-)-(6R)-massoialactone, 1991 ; Pirkle et al., Enantiomerically pure lactones, 1980 ; document brevet JP02059564; Hoeyer et al., A convenient synthesis of homochiral delta alkylated alpha,beta unsaturated delat-lactone, 1991 ; Fehr et al., Novel approach to the synthesis of 6-substitutated 5,6-dihydro-2(2H)-pyranones, 1981 ; et Pan et al., An efficient and stereoselective synthesis of (-)-massoialactone, 1996 ; ou encore JPH0259564).

Toutefois, il est important de noter que l'odeur de la massoia lactone diffère entre la forme (R)-(-)-massoia lactone et (S)-(+)-massoia lactone. En effet, la forme (S)-(+)-massoia lactone présente une odeur de beurre alors que c'est la forme (R)-(-)-massoia lactone qui présente un parfum caractéristique de lait de noix de coco (Nohira et al., Optical Resolution of Fragrant Lactones, 2000). Par conséquent, de manière à répondre aux besoins, il est indispensable d'utiliser un procédé permettant de fabriquer la forme (R)-(-)-massoia lactone avec une grande pureté optique.

Hors, il est relativement coûteux de développer et d'utiliser de tels procédés de synthèse en raison notamment des contraintes liées au choix des matériaux de départ et à chacune des étapes pour obtenir la forme (R)-(-)-massoia lactone avec une grande pureté optique.

De plus, l'obtention par synthèse chimique répond de moins en moins aux demandes des consommateurs.

Une solution alternative s'est imposée : la production de lactones en général, et plus particulièrement de (R)-(-)-massoia lactone, par voie biotechnologique. Il est possible par exemple d'utiliser des cellules microbiennes et des enzymes pour biotransformer ou bioconvertir des acides gras en lactones. Différentes études et des documents brevets ont été publiés sur ce sujet.

On connait le document brevet JP3779751 qui divulgue un procédé de fabrication simple de (R)-(-)-massoia lactone à partir de la culture d'un micro-organisme, et plus particulièrement du micro-organisme marin *Exophiala pisciphila* N1-10102 (déposé auprès de l'Institut de Recherche sur la Fermentation (Agence des Sciences et Technologies Industrielles) au Japon, sous le numéro P-14232). Un tel procédé permet de générer de la (R)-(-)-massoia lactone dans le milieu réactionnel et de récupérer la (R)-(-)-massoia lactone au moyen d'une étape de séparation. Le milieu de culture utilisé comprend une source d'azote telle que la L-asparagine, une source de carbone telle que le glucose et des minéraux tels que le chlorure de sodium, le chlorure de potassium, le dihydrogénophosphate de potassium mais ne comprend pas de calcium, et après 5-10 jours de culture à 22-27°C à pH 6-7, le milieu est acidifié par un acide tel que l'acide sulfurique en présence d'un solvant tel que le méthanol et chauffé pour obtenir la (R)-(-)-massoia lactone dans le milieu réactionnel.

Aussi, le rendement obtenu divulgué dans ce document est de seulement 1,4 mg/100 mL soit 14 mg/L.

On connait également la publication Price et al. (Structural characterization of novel extracellular liamocins (mannitol oils) produced by Aureobasidium pullulans strain NRRL 50380, 2013) qui divulgue un procédé d'obtention de massoia lactone à partir de liamocines extraites de la culture de la souche *d'Aureobasidium pullulans* NRRL 50380 dans un milieu contenant 5% (poids/volume) de sucrose. Les liamocines sont extraites en utilisant une méthyl éthyl cétone et le rendement maximal en liamocines est obtenu au jour 6 avec un rendement compris entre 0,5 et 6 g d'huile de mannitol (liamocines)/L. La massoia lactone est alors obtenue après méthanolyse avec du méthanol anhydre en présence d'acide chlorhydrique, centrifugation de manière à éliminer un lourd précipité blanc et récupérer la fraction soluble au méthanol comprenant la massoia lactone.

En outre, on connait la publication Kurosawa et al. (Extracellular accumulation of the polyol lipids, 3,5-dihydroxydecanoyl and 5-hydroxy-2-decenoyl esters of arabitol and mannitol, by Aureobasidium sp. 1994). Cette publication divulgue que de nombreuses souches d'*Aureobasidium pullulans* produisent de grandes quantités de poly(β-L-acide malique) dans un milieu contenant du carbonate de calcium CaCO₃. Cette publication révèle qu'en absence de CaCO₃ dans le milieu de culture, ces souches produisent des lipides extracellulaires tels que des huiles lourdes au lieu des poly(acide malique). Cette publication décrit plus particulièrement l'obtention de (+)-3-hydroxydecan-5-olide et R-(-)-massoia lactone à partir de la culture de la souche d'*Aureobasidium pullulans* A-21M pendant 7 jours à 25°C dans un milieu contenant 12% de glucose, 0,15% NaNO₃, 0,10% KNO₃, 0,005% KH₂PO₄, 0,02% MgSO₄ 7H₂O, 2 ppm ZnSO₄ 7H₂O et 0,02% d'extrait de levure dans de l'eau déminéralisée, ce milieu ne comprenant donc pas de CaCO₃. 35 g d'huiles lourdes comprenant des lipides sont extraits avec la méthyl éthyl cétone. Environ 10 g de lipides sont saponifiés par ajout d'hydroxyde de sodium NaOH suivie d'une hydrolyse par ajout d'acide sulfurique H₂SO₄ pour récupérer environ 4 g d'acides gras. Les composés (+)-3-hydroxydecan-5-olide et R-(-)-massoia lactone sont enfin récupérés par extraction à l'éther.

Toutefois, on peut en déduire que le rendement en R-(-)-massoia lactone ainsi obtenu est faible au regard des 35 g d'huiles lourdes comprenant des lipides isolés selon ce procédé. Sur la base des résultats mentionnés dans ce document, le rendement en R-(-)-massoia lactone est de 1,3 g/L.

Considérant ce qui précède, un problème que se propose de résoudre l'invention est de développer un procédé alternatif de production de lactones, plus particulièrement de (R)-(-)-massoia lactone qui soit utilisable en tant que telle, à partir d'une souche d'*Aureobasidium pullulans,* qui soit facile à mettre en oeuvre, peu coûteux et qui présente une bonne reproductibilité, à partir de ressources durables et naturelles, et une productivité améliorée (quantité de lactone d'intérêt formée et vitesse de production améliorées).

L'invention a donc pour objet un procédé de production de lactones à partir d'une souche d'*Aureobasidium pullulans,* caractérisé en ce qu'il comprend les étapes suivantes selon lesquelles :
- on réalise une préculture de la souche *Aureobasidium pullulans* CBS 771.97 (obtenue auprès du CBS-KNAW Fungal Biodiversity Centre, Uppsalalaan 8, 3584 CT Utrecht, Pays-Bas) ou d'une des souches *Aureobasidium pullulans* qui appartient au même clade que la souche CBS 771.97;
- à partir d'un inoculum issu de la préculture, on réalise une culture par fermentation à une température comprise entre 20°C et 40°C pendant une période d'au moins 3 jours de manière à produire des métabolites, dans un milieu de production aqueux stérilisé contenant :
   - une source de carbone ;
   - une source d'azote ;
   - une solution de sels minéraux ; et
   - une source de calcium, à une concentration comprise entre 2 et 100 mM ; et
- à l'issue de la période de fermentation, on transforme les métabolites produits en un mélange de lactones comprenant la (R)-5,6-dihydro-6-pentyl-2H-pyran-2-one et/ou la (4R,6R)-4-hydroxy-6-pentyl-tetrahydro-2H-pyran-2-one.

L'invention et les avantages qui en découlent seront mieux compris à la lecture de la description et des modes de réalisation non limitatifs qui suivent, au regard des figures annexées dans lesquelles :
- la figure 1 représente un arbre phylogénétique (construit avec ClustalW™ et iTol™) du genre *Aureobasidium* basé sur les séquences partielles du gène de la béta-tubuline déposées dans la base de données GenBank® ou séquencées par le Demandeur ;
- la figure 2 est un histogramme représentant le suivi de la production de lactones selon un exemple du procédé selon l'invention par différentes souches d'*Aureobasidium* en culture à 25°C ;
- la figure 3 est un histogramme représentant le suivi de la production de lactones selon un même exemple du procédé selon l'invention par différentes souches d'*Aureobasidium* en culture à 30°C ;
- la figure 4 est un graphique représentant l'effet de la température de l'étape de culture sur la production de lactones selon un exemple du procédé selon l'invention pour la souche *Aureobasidium pullulans* CBS 771.97 ;
- la figure 5 est un graphique représentant l'effet de la source de carbone sur la production de lactones selon un exemple du procédé selon l'invention pour la souche *Aureobasidium pullulans* CBS 771.97 ;
- la figure 6 est un graphique représentant le suivi de la production de lactones, de la productivité et de la consommation de glucose selon un exemple du procédé selon l'invention pour la souche *Aureobasidium pullulans* CBS 771.97 ; et
- la figure 7 est un graphique représentant l'effet de l'ajout séquentiel de glucose dans le milieu au cours de l'étape de culture sur la production de lactones et la productivité selon un exemple du procédé selon l'invention pour la souche *Aureobasidium pullulans* CBS 771.97.

Dans cette description, à moins qu'il ne soit spécifié autrement, il est entendu que, lorsqu'un intervalle est donné, il inclut les bornes supérieure et inférieure dudit intervalle.

Le procédé selon l'invention est mis en oeuvre pour la production de lactones à partir de la souche *Aureobasidium pullulans* CBS 771.97 ou d'une des souches *Aureobasidium pullulans* qui appartient au même clade que la souche CBS 771.97.

Le procédé mis en oeuvre selon l'invention a été développé de manière à permettre la reproductibilité des cultures et des analyses des métabolites produits.

*Aureobasidium pullulans* est un champignon ubiquitaire polymorphique de type ascomycète. Ce champignon, souvent présenté comme une levure noire, présente un cycle de reproduction complexe, comprenant des formes unicellulaires variées (cellules de type levure, blastospores, chlamydospores) et une forme mycélienne filamenteuse au cours de laquelle les hyphes individuels produisent des cellules unicellulaires par bourgeonnement. Ces différents états physiologiques coexistent en proportion variée en fonction des conditions de culture (sources de carbone et d'azote, pH, concentration en oxygène et en sels minéraux,...).

Le Demandeur a cherché à maitriser le cycle de reproduction d'*Aureobasidium pullulans,* et plus particulièrement de la souche *Aureobasidium pullulans* CBS 771.97 (obtenue auprès du CBS-KNAW Fungal Biodiversity Centre, Uppsalalaan 8, 3584 CT Utrecht, Pays-Bas) ou ses souches apparentées.

Par souche apparentée à la souche *Aureobasidium pullulans* CBS 771.97 selon l'invention, on entend une souche *Aureobasidium pullulans* qui appartient au même clade que la souche CBS 771.97. D'un point de vue phylogénétique, un clade se définit par un ensemble d'organismes formant un groupe monophylétique, c'est à dire présentant un ancêtre commun et regroupant tous ses descendants. Ainsi, des espèces d'un même clade seront toujours plus proches entre elles que d'une autre espèce extérieure à ce clade. Les représentants d'un même clade présentent au moins un caractère propre à tout le groupe et qui a été hérité d'un même ancêtre commun. La répartition des souches d'*Aureobasidium* dans différents clades est basée sur le pourcentage de similarité de la séquence de leur gène de béta-tubuline (BT2) ainsi que cela a été défini par Manitchotpisit et al. (Multilocus phylogenetic analyses, pullulan production and xylanase activity of tropical isolates of Aureobasidium pullulans, 2009; Heavy oils produced by Aureobasidium pullulans, 2011 ; et Poly(β-L-malic acid) production by diverse phylogenetic clades of *Aureobasidium pullulans,* 2012). Ainsi, les souches d'*Aureobasidium* appartenant à un même clade présentent des similarités de séquences du gène des béta-tubulines plus élevées entre elles que celles appartenant à un autre clade.

Dans le cadre de l'invention, nous appliquons les critères définis par Manitchotpisit et al. pour déterminer les souches apparentées et selon cette classification, la souche CBS 771.97 appartient donc au clade N°7.

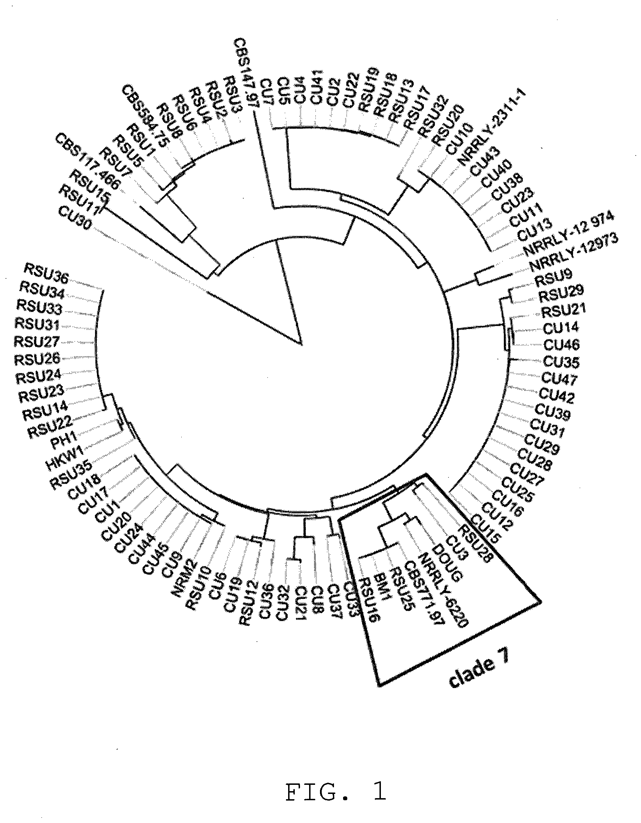

A titre d'exemple non limitatif de souche apparentée à la souche CBS 771.97 appartenant au clade N°7, on peut citer les souches du genre *Aureobasidium* RSU28, CU3, DOUG, NRRLY-6220, RSU25, BM1, RSU16 tel que représenté à la figure 1.

Selon l'invention, il est particulièrement avantageux d'obtenir la forme levure qui permet de travailler dans un milieu plus homogène et donc plus favorable à une bonne reproductibilité. La forme filamenteuse est beaucoup plus délicate à gérer ce qui a un impact important sur la reproductibilité des fermentations. De plus, la forme filamenteuse ne produit que peu voire pas de métabolites précurseurs de lactones.

Une étape de préculture a ainsi été introduite afin de pouvoir partir d'un lot (inoculum) d'une même quantité de cellules dans le même état physiologique pour chaque culture. En effet, il est quasiment impossible d'obtenir des croissances synchrones en une seule étape. Bien que les étapes de précultures soient des pratiques relativement classiques en microbiologie, les durées respectives de chaque étape ainsi que le choix du milieu de préculture et la température ont été adaptés à la souche *Aureobasidium pullulans* CBS 771.97 ou ses souches apparentées, utilisées selon l'invention afin de contrôler la forme sous laquelle elle se développe, préférentiellement la forme levure par rapport à la forme filamenteuse.

Ainsi, dans une première étape du procédé selon l'invention, on réalise une préculture de la souche *Aureobasidium pullulans* CBS 771.97 ou ses souches apparentées, à partir par exemple d'un cryotube de cette souche conservée à -80°C.

L'étape de préculture est préférentiellement réalisée dans un milieu solide et/ou liquide choisi parmi un milieu à base d'extrait de malt, par exemple Malt Extract Fluka™ n°70167 (Sigma-Aldrich) ou Malt Extract™ (MP Biomedicals), et avantageusement de peptones, par exemple Bacto™ peptone (Difco), ou un milieu YNB (« Yeast Nitrogen Base »), par exemple Difco™ Yeast Nitrogen Base.

Préférentiellement, l'étape de préculture comprend au moins une première étape de préculture en milieu solide de manière à contrôler l'état de développement du micro-organisme et assurer la reproductibilité, et au moins une seconde étape de préculture en milieu liquide pour permettre au micro-organisme de s'adapter à un milieu liquide et de contrôler la forme du micro-organisme (en particulier levure) et la production de cellules pour la culture.

A titre d'exemple, l'étape de préculture comprend avantageusement les étapes suivantes selon lesquelles :
- à partir d'un cryotube de la souche *Aureobasidium pullulans* CBS 771.97 ou ses souches apparentées, on ensemence un tube de gélose à l'extrait de malt et de peptones, incubé à 25°C jusqu'à l'apparition de colonies ;
- à partir de ce tube, on ensemence par strie une boite de Pétri contenant une gélose à l'extrait de malt et de peptones, incubée à 25°C pendant 24h ; et
- après croissance sur boite, on ensemence un clone dans un tube d'extrait de malt liquide, incubé à 25°C sous une agitation de 200 tours/minute pendant environ 18h.

Cette étape avantageuse de préculture sert ensuite à l'ensemencement de fiole ou de bioréacteur/fermenteur pour l'étape de culture par fermentation de la souche *Aureobasidium pullulans* CBS 771.97 ou ses souches apparentées dans un milieu de production liquide et dans des conditions définis selon l'invention.

Bien entendu, dans la mesure où l'étape de culture est réalisée dans un bioréacteur/fermenteur d'1 litre à plus de 1000 litres qui permet d'optimiser le contrôle des conditions de culture et d'augmenter les quantités de métabolites produites, les étapes de préculture peuvent être multipliées de manière à obtenir les quantités/volumes d'inoculum souhaités.

A partir d'un inoculum issu de la préculture, on réalise alors une culture par fermentation à une température comprise entre 20°C et 40°C pendant une période d'au moins 3 jours de manière à produire des métabolites, plus particulièrement des sucrolipides, dans un milieu de production aqueux stérilisé contenant une source de carbone, une source d'azote, une solution de sels minéraux, et une source de calcium, et éventuellement une source de vitamine.

Préférentiellement, le volume de préculture de la souche *Aureobasidium pullulans* CBS 771.97 ou ses souches apparentées inoculé dans le milieu de production liquide est relatif à une Densité Optique (DO) initiale du milieu de culture, comprenant le milieu de production et l'inoculum, comprise entre 0,5 et 2, encore plus préférentiellement une DO initiale de 0,5.

Le volume de l'inoculum est préférentiellement compris entre 3 et 10% du volume du milieu de culture.

L'étape de culture par fermentation est préférentiellement réalisée à une température de 25°C à 35°C, encore plus préférentiellement entre 28°C et 30°C, par exemple à 30°C.

De plus, l'étape de culture par fermentation est préférentiellement réalisée sous agitation pendant une période de 3 à 20 jours, plus préférentiellement de 5 à 18 jours, encore plus préférentiellement de 7 à 15 jours, par exemple 7 jours, 10 jours, 12 jours, 14 jours ou encore 15 jours.

A titre d'exemple illustratif, lorsque la fermentation est réalisée en fiole bafflée de 300 mL remplie à 10% de milieu de production, l'agitation est constante, de l'ordre de 140 tours/minute. En fermenteur, l'agitation peut être par exemple de l'ordre de 500 tours/minute.

Avantageusement, l'aération a également une influence, et peut être par exemple de l'ordre de 0,5 vvm lors de culture en bioreacteur/fermenteur.

Toutefois, les vitesse d'agitation et l'aération choisies dépendent de la géométrie de chaque fermenteur. Ainsi, en fermenteur/bioréacteurs, l'homme du métier veillera à ajuster les paramètres d'aération et d'agitation afin d'avoir une oxygénation au moins équivalente à celle d'une fiole.

Le milieu de production aqueux stérilisé utilisé dans l'étape de culture par fermentation de la souche *Aureobasidium pullulans* CBS 771.97 ou ses souches apparentées selon l'invention contient une source de carbone, une source d'azote, une solution de sels minéraux, une source de calcium, et éventuellement une source de vitamine.

La fiole ou le bioréacteur/fermenteur est rempli par le milieu de production aqueux stérilisé, puis par l'inoculum pour former le milieu de culture.

Selon un mode de réalisation du procédé selon l'invention, la fermentation se déroule ensuite sans addition supplémentaire de milieu de production.

Selon un mode de réalisation avantageux du procédé selon l'invention, on ajoute de manière séquentielle du milieu de production, et plus particulièrement la source de carbone, au cours de l'étape de culture pour maintenir et optimiser la productivité.

Le milieu de production aqueux utilisé dans l'étape de culture selon l'invention est préférentiellement préparé dans de l'eau ultrapure de conductivité comprise entre 15 et 18 MégaOhms, avantageusement 18 MégaOhms.

Le milieu de production utilisé dans l'étape de culture selon l'invention est également stérilisé de manière à réduire le risque de contaminations.

La source de carbone est préférentiellement choisie parmi le glucose, le mannose, le saccharose, le xylose, le maltose, le fructose et le glycérol, pris seul ou en combinaison, préférentiellement le glucose.

La source de carbone est préférentiellement utilisée à une concentration de 30 à 150 g/L, par exemple 45, 60, 75, 90 ou 120 g/L, encore plus préférentiellement à 60 g/L pour le glucose.

La source d'azote est préférentiellement choisie parmi le nitrate d'ammonium NH₄NO₃, le nitrate de sodium NaNO₃, le sulfate d'ammonium (NH₄)₂SO₄, des peptones (par exemple Bacto™ peptone (Difco)) ou un acide aminé naturel, pris seul ou en combinaison, préférentiellement le nitrate d'ammonium NH₄NO₃.

La source d'azote est préférentiellement utilisée à une concentration de 0,1% à 1% (poids/volume), par exemple 0,1%, 0,2% ou 0,5%, encore plus préférentiellement 0,1% de NH₄NO₃.

Les sels minéraux en solution sont préférentiellement choisis parmi les ions magnésium, phosphate, potassium, sodium, chlorure, sulfate, pris seul ou en combinaison.

A titre d'exemple illustratif, la solution de sels minéraux est un mélange de KH₂PO₄, KCI, MgSO₄ et HCl.

Le milieu de production aqueux stérilisé défini et utilisé dans l'étape de culture selon l'invention contient également une source de calcium à une concentration dans le milieu de production comprise entre 2 et 100 mM, préférentiellement de 50 mM.

La source de calcium est préférentiellement choisie parmi du carbonate de calcium CaCO₃ précipité pur à au moins 98% et comportant au moins un oligo-élément, solubilisé en condition acide et rajouté à la solution de sels minéraux extemporanément, ou du chlorure de calcium CaCl₂ en présence d'une solution d'oligo-élément(s). Dans la mesure où le CaCO₃ est utilisé pur, par exemple à 100%, et ne comporte pas d'oligo-élément, il est utilisé en combinaison avec au moins un oligo-élément.

L'oligo-élément associé à la source de calcium est préférentiellement choisi parmi le manganèse, cuivre, cobalt, fer, zinc, strontium, plomb, mercure, chlore, bore, molybdène et soufre, par exemple le fer et/ou le manganèse, pris seul ou en combinaison.

La concentration en oligo-élément(s), pris seul ou en combinaison, dans le milieu de production est préférentiellement d'au plus de l'ordre de 0,1 mM.

Une différence majeure et essentielle avec l'art antérieur, outre le choix spécifique de la souche d'intérêt *Aureobasidium pullulans* CBS 771.97 ou ses souches apparentées, est l'utilisation dans le milieu de production d'une source de calcium avantageuseument sous forme de CaCO₃ ou CaCl₂ en présence d'au moins un oligo-élément, préférentiellement le manganèse. En effet, contrairement à l'art antérieur cité dans lequel la présence de CaCO₃ augmente la production d'acide polymalique mais diminue la quantité de lipides d'intérêt produits, dans le procédé selon l'invention, le Demandeur a mis en évidence le rôle essentiel et inattendu de ce composé dans la production des métabolites et plus particulièrement de sucrolipides, et par conséquent de lactones, et plus particulièrement de la (R)-5,6-dihydro-6-pentyl-2H-pyran-2-one (ou (R)-(-)-massoia lactone) et/ou d'une hydroxylactone, à savoir la (4R,6R)-4-hydroxy-6-pentyl-tetrahydro-2H-pyran-2-one.

L'effet du calcium est donc inattendu dans le procédé selon l'invention, et inverse à celui connu du CaCO₃ dans l'art antérieur.

Selon un exemple de mode de réalisation avantageux selon l'invention, une étude des concentrations optimales pour le CaCO₃ précipité (Rectapur™ Prolabo n°22296.294) de pureté supérieure ou égale à 98% a permis de déterminer qu'à faible concentration (0,02%), il permet déjà une amélioration du rendement et qu'à plus haute concentration (0,5%), il est en plus probablement responsable de la décantation de la phase huileuse d'intérêt comprenant les précurseurs de lactones transformés ensuite en (R)-5,6-dihydro-6-pentyl-2H-pyran-2-one et/ou (4R,6R)-4-hydroxy-6-pentyl-tetrahydro-2H-pyran-2-one. La présence de ces précurseurs a été montrée. Il est possible que la lactone soit aussi formée spontanément en petite quantité, en particulier dans le cas d'un stockage prolongé.

Avantageusement, le milieu de production contient en outre une source de vitamine.

La source de vitamine est préférentiellement choisie parmi un extrait de levure, par exemple Bacto™ Yeast extract (Difco) ou un complexe de vitamine B.

A titre d'exemple non limitatif de milieu de production particulièrement préféré selon l'invention dans lequel est inoculé un volume de la préculture d'*Aureobasidium pullulans* CBS 771.97 ou ses souches apparentées :
- la source de carbone est du glucose à 60 g/L préparé dans de l'eau ultrapure et stérilisée à 115°C ;
- la source d'azote et la solution de sels minéraux sont préparées dans de l'eau ultrapure et stérilisées à 120°C et contiennent un mélange de 1 g/L de NH₄NO₃, 0,1 g/L de KH₂PO₄, 0,5 g/L de KCl, 0,2 g/L de MgSO₄, 7H₂O, et en outre 0,5 g/L d'extrait de levures comme source de vitamine (Bacto™ Yeast extract (Difco)) ; et
- le CaCO₃ (Rectapur™ Prolabo n°22296.294) à 0,5% (poids/volume) est solubilisé dans de l'eau ultrapure acidifiée par de l'acide chlorhydrique, à un pH d'environ 5, puis est ajouté à la solution de sels minéraux après stérilisation par autoclavage.

Selon un autre exemple, dans le cas où la source de calcium est du CaCl₂ en solution avec des oligo-éléments, elle est préparée dans de l'eau ultrapure et stérilisée par filtration à travers un filtre stérile de 0,2 µm.

Cette étape de culture en milieu liquide permet ainsi la production de biomasse (cellules) et de métabolites.

Les métabolites produits pendant l'étape de culture sont essentiellement des sucrolipides. Le Demandeur a notamment identifié la production de sucrolipides à base de monomère de l'acide dihydroxydécanoïque, et plus particulièrement la production de 21 métabolites, notamment l'halymécine, l'halymécine-arabitol, l'halymécine-mannitol et l'exophiline-mannitol.

Dans le procédé selon l'invention, à l'issue de la période de fermentation, on transforme les métabolites ainsi produits en un mélange de lactones comprenant la (R)-5,6-dihydro-6-pentyl-2H-pyran-2-one et/ou la (4R,6R)-4-hydroxy-6-pentyl-tetrahydro-2H-pyran-2-one. Plus particulièrement, on transforme les métabolites produits en un mélange de lactones en hydrolysant en milieu acide ou basique, ou encore par hydrolyse enzymatique, les sucrolipides produits en monomères, et en les cyclisant pour obtenir un mélange de lactones comprenant la (R)-5,6-dihydro-6-pentyl-2H-pyran-2-one et/ou la (4R,6R)-4-hydroxy-6-pentyl-tetrahydro-2H-pyran-2-one qui sont dosables par chromatographie en phase gazeuse.

Il convient de traiter l'ensemble du milieu de culture pour obtenir les lactones cibles de manière à éviter des problèmes en termes de reproductibilité des résultats en raison de l'hétérogénéité du milieu qui augmente avec la quantité de sucrolipides produits dans la mesure où un aliquot est seulement prélevé.

De manière alternative, on peut avantageusement centrifuger l'ensemble du milieu de culture de manière à éliminer le surnageant et à traiter le culot ainsi récupéré contenant les cellules et les sucrolipides produits pour obtenir les lactones cibles. En effet, la transformation est ainsi séparée de la production de sucrolipides et ces étapes peuvent donc être avantageusement effectuées sur 2 sites différents. En outre, les volumes à traiter sont plus petits et nécessitent ainsi un appareillage moins encombrant.

Selon un premier mode de transformation des métabolites produits, on réalise, à l'issue de la période de fermentation, sur l'ensemble du milieu de culture ou avantageusement sur le culot après centrifugation, une saponification des sucrolipides, par exemple par ajout d'hydroxyde de sodium NaOH 4N, suivie par une acidification, par exemple par ajout d'acide sulfurique H₂SO₄ 50% et chauffage, par exemple à 90°C pendant 4h. Un mélange de (R)-5,6-dihydro-6-pentyl-2H-pyran-2-one (ou (R)-(-)-massoia lactone) et d'une hydroxylactone, à savoir la (4R,6R)-4-hydroxy-6-pentyl-tetrahydro-2H-pyran-2-one, est obtenu dont les proportions varient selon la concentration en acide, la température et la durée de chauffage. Le ratio (R)-(-)-massoia lactone / hydroxylactone augmente quand on augmente la concentration en acide, la température de chauffage et/ou la durée. Aussi, de l'hydroxylactone reste en général présente à l'issue du traitement et pourra être avantageusement déshydratée en (R)-5,6-dihydro-6-pentyl-2H-pyran-2-one d'intérêt.

Selon un deuxième mode avantageux de transformation des métabolites produits, à l'issue de la période de fermentation, on acidifie, par exemple par ajout d'acide sulfurique H₂SO₄ 50% (normalité finale 1,2 N), d'acide citrique ou d'acide tartrique, et on chauffe, par exemple entre 90°C et 110°C pendant 12h à 24h, directement le milieu de culture. L'hydrolyse des sucrolipides (formation des monomères) est concommitamment suivie de la lactonisation et de la déshydratation des monomères formés pour obtenir la (R)-5,6-dihydro-6-pentyl-2H-pyran-2-one (ou (R)-(-)-massoia lactone) d'intérêt. Les 3 réactions sont faites simultanément. De l'hydroxylactone peut être présente si les conditions de réaction (température, normalité acide, durée) ne sont pas suffisantes. Le ratio (R)-(-)-massoia lactone / hydroxylactone augmente quand on augmente la concentration en acide, la température de chauffage et/ou la durée. Si de l'hydroxylactone reste présente à l'issue du traitement, elle pourra être avantageusement déshydratée en (R)-(-)-massoia lactone d'intérêt. Encore plus avantageusement, ce mode de transformation est appliqué sur le culot.

Selon un troisième mode de transformation des métabolites produits, à l'issue de la période de fermentation, on extrait le milieu de culture ou le culot obtenu après centrifugation avec un solvant. On utilise par exemple l'acétate d'éthyle tant qu'on n'acidifie pas le milieu, les sucrolipides étant stables dans l'acétate d'éthyle mais pas les lactones. Le solvant est ensuite évaporé et à partir des sucrolipides extraits, on réalise alors une saponification, acidification et chauffage selon le premier mode de réalisation ou directement une acidification et chauffage selon le deuxième mode de réalisation. On peut également réaliser une transformation thermique à 250°C environ.

D'autres modes de transformation peuvent également être envisagés. Par exemple, on peut extraire le culot contenant les métabolites produits au CO₂ supercritique et obtenir directement la (R)-5,6-dihydro-6-pentyl-2H-pyran-2-one (ou (R)-(-)-massoia lactone) d'intérêt.

Ainsi, dans la mesure où, selon les conditions de transformation choisies, on obtient un mélange de (R)-5,6-dihydro-6-pentyl-2H-pyran-2-one et de (4R,6R)-4-hydroxy-6-pentyl-tetrahydro-2H-pyran-2-one, le procédé selon l'invention comprend en outre une étape selon laquelle on transforme l'hydroxylactone obtenue en (R)-5,6-dihydro-6-pentyl-2H-pyran-2-one d'intérêt en la déshydratant, par exemple en présence d'un acide après l'avoir solubilisée dans un solvant organique.

Enfin, on isole la (R)-5,6-dihydro-6-pentyl-2H-pyran-2-one d'intérêt obtenue par extraction par un solvant organique tel que par exemple le méthyl t-butyl éther, la méthyl éthyl cétone, l'acétate d'éthyle, le chloroforme, le dichlorométhane et le carbonate de diméthyle suivie d'une distillation, ou par extraction au CO₂ supercritique, ou par hydrodistillation.

On récupère ainsi la (R)-5,6-dihydro-6-pentyl-2H-pyran-2-one (ou (R)-(-)-massoia lactone) d'intérêt, utilisable en tant que telle.

Le procédé selon l'invention permet ainsi l'obtention de (R)-(-)-massoia lactone présentant un parfum caractéristique de lait et de pulpe de noix de coco fidèle à celui de la (R)-(-)-massoia lactone obtenue à partir de l'huile d'écorce de l'arbre *Cryptocaria massoia.* La (R)-(-)-massoia lactone produite par le procédé selon l'invention est ainsi utilisable en tant que telle comme arôme ou parfum dans des produits alimentaires, des boissons, et autres. De plus, elle est produite par voie biotechnologique selon le procédé selon l'invention à bas coût, en grande quantité apte à répondre à la demande du marché et selon un procédé qui respecte les contraintes environnementales.

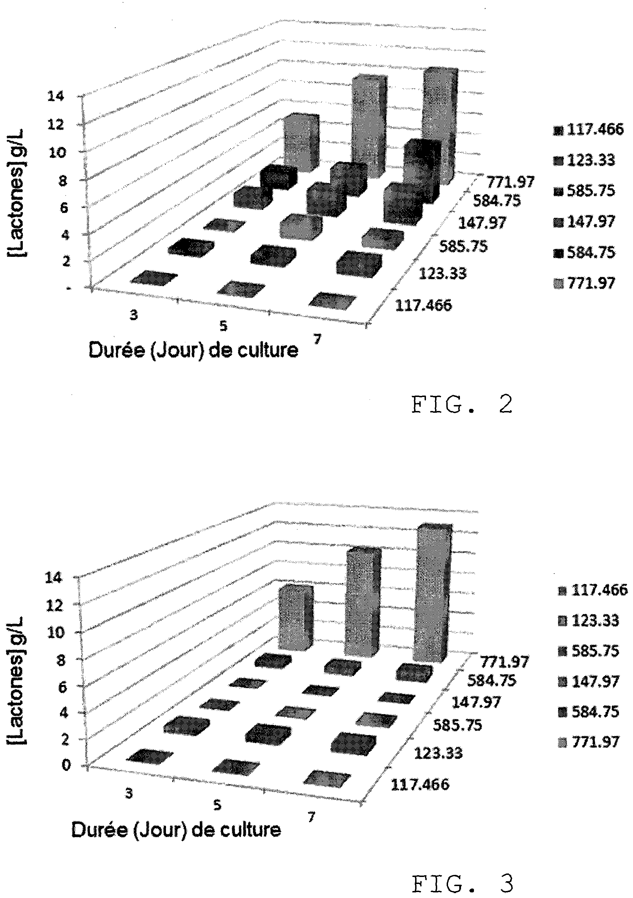

Les avantages du procédé de production de lactones, et notamment de (R)-5,6-dihydro-6-pentyl-2H-pyran-2-one (ou (R)-(-)-massoia lactone), selon l'invention sont mis en évidence par les résultats des exemples ci-après et illustrés par les figures 2 à 7.

### Exemple 1 :

A titre d'exemple illustratif du procédé de production selon l'invention, le Demandeur a criblé dans une étude comparative différentes souches d'*Aureobasidium* de clades différents, selon les conditions opératoires de production mises au point selon l'invention permettant d'obtenir une bonne reproductibilité afin de déterminer la productivité en lactones par les différentes souches disponibles testées.

Le Demandeur a ainsi acheté plusieurs souches d'*Aureobasidium,* tel que répertorié dans le tableau 1 ci-dessous.

**Tableau 1 : Récapitulatif de l'ensemble des souches testées**

| **Identifiant Souche** | **Nom** |
|---|---|
| CBS 771.97 | *Aureobasidium pullulans var. pullulans* |
| CBS 585.75 | *Aureobasidium pullulans var. pullulans* |
| CBS 584.75 | *Aureobasidium pullulans var. pullulans* |
| CBS 123.33 | *Exophiala lacanii-corni (A.pullulans* jusqu'en 1967) |
| CBS 117.466 | *Aureobasidium pullulans* |
| CBS 147.97 | *Aureobasidium pullulans var. namibiae* |

Les conditions opératoires de l'exemple du procédé selon l'invention réalisées dans cette étude sont plus particulièrement décrites ci-dessous et sont identiques pour l'ensemble des souches criblées.

On réalise une préculture à partir d'un cryotube de chacune des souches criblées selon les étapes suivantes :
- étape 1 : on ensemence un tube de gélose à base d'extrait de malt et de peptones (milieu ME solide : MEA Fluka™) incubé à 25°C avec croissance jusqu'à l'apparition de colonies ;
- étape 2 : à partir de ce tube, on ensemence par strie une boite de Pétri contenant un milieu ME solide (MEA Fluka™), et on incube la boite à 25°C pendant 24h ; et
- étape 3 : après croissance sur boite, on ensemence un clone dans un tube de 5 mL à base d'extrait de malt liquide (milieu ME liquide), incubé à 25°C sous une agitation de 200 tours/minute pendant 18h.

Le lendemain, on rassemble l'ensemble des tubes contenant une même souche et on fait une lecture de la Densité Optique (DO).

La préculture ainsi réalisée sert alors à l'ensemencement de fioles pour l'étape suivante de culture par fermentation. Dans cette étude, la taille de l'inoculum est fixée de manière à avoir une valeur de DO initiale de la culture de 0,5.

En fonction de la croissance de chacune des souches criblées, le volume de préculture inoculé par fiole varie légèrement tel qu'illustré dans le tableau 2 ci-dessous :

**Tableau 2 : Volume de préculture nécessaire pour chaque souche testée**

| **Souche** | **DO 600 nm** | **V ensemencement pour DO**ᵢₙᵢₜᵢₐₗₑ **= 0,5** |
|---|---|---|
| CBS 771.97 | 5 | 3 mL |
| CBS 585.75 | 7,6 | 2 mL |
| CBS 584.75 | 7,6 | 2 mL |
| CBS 123.33 | 8,7 | 1,7 mL |
| CBS 117.466 | 9,5 | 1,7 mL |
| CBS 147.97 | 5 | 3 mL |

Ainsi, à partir de la préculture réalisée pour chaque souche, on ensemence le volume de préculture déterminé pour chaque souche (cf. tableau 2) dans des fioles bafflées de 300 mL contenant 30 mL du milieu de production qui comprend :
- une source de carbone constituée de glucose à 60 g/L préparé dans de l'eau ultrapure et stérilisé séparément à 115°C ;
- une source d'azote et une solution de sels minéraux préparées dans de l'eau ultrapure et stérilisées à 120°C comprenant:
   NH₄NO₃: 1 g/L;
   KH₂PO₄: 0,1 g/L;
   KCl: 0,5 g/L;
   MgSO₄, 7H₂O: 0,2 g/L; et
- un extrait de levure 0,5 g/L (Bacto™ Yeast extract (Difco)) comme source de vitamine ; et
- 5 g/L de carbonate de calcium CaCO₃ précipité (Rectapur™ Prolabo n°22296.294) solubilisé dans de l'eau ultrapure acidifiée par de l'acide chlorhydrique, à un pH de l'ordre de 5, et rajouté à la solution de sels extemporanément.

L'étape de culture des différentes souches criblées est alors réalisée à deux températures de fermentation, 25°C et 30°C.

Les fioles sont donc incubées en parallèle à 25 et 30°C, sous agitation (140 tours/minute).

Les fioles sont arrêtées à 3, 5 et 7 jours de culture afin de déterminer le rendement en lactones totales produites, à savoir un mélange de (R)-5,6-dihydro-6-pentyl-2H-pyran-2-one et/ou de (4R,6R)-4-hydroxy-6-pentyl-tetrahydro-2H-pyran-2-one, pour chaque souche testée.

Le milieu de fermentation de chaque fiole est alors saponifié sur la nuit par ajout de NaOH 4N, puis acidifié par H₂SO₄ 50% et enfin chauffé à 90°C pendant 1h.

Le mélange de lactones ainsi produites est récupéré par extraction au methyl *t*-butyl ether.

La phase organique est injectée en chromatographie en phase gazeuse pour une analyse quantitative des lactones ainsi produites.

L'ensemble des résultats obtenus pour le criblage des différentes souches d'*Aureobasidium* dans le cadre de cette étude est reporté dans le tableau 3 ci-dessous.

**Tableau 3 : Suivi du rendement total en lactones des différentes souches criblées selon le procédé selon l'invention à 25°C et 30°C**

| **Identifiant Souche** | **25°C (g/L)** | | | **30°C (g/L)** | | |
|---|---|---|---|---|---|---|
| | **J3** | **J5** | **J7** | **J3** | **J5** | **J7** |
| CBS 771.97 | 5,2 | 9,5 | 10,6 | 6,1 | 10,3 | 13,0 |
| CBS 585.75 | ND | 1,2 | 0,8 | 0 | 0,04 | 0,2 |
| CBS 584.75 | 1,5 | 2,4 | 5,2 | 0,58 | 0,6 | 0,8 |
| CBS 123.33 | 0,45 | 0,6 | 0,9 | 0,51 | 0,6 | 0,7 |
| CBS 117.466 | 0 | 0 | 0 | 0 | 0 | 0 |
| CBS 147.97 | 1,2 | 2,2 | 2,8 | 0 | 0 | 0 |

| | | | | | | |
|---|---|---|---|---|---|---|
| ND : Non déterminé | | | | | | |

Les résultats de l'étude comparative répertoriés dans le tableau 3 ci-dessus sont plus particulièrement illustrés par les figures 2 et 3.

La figure 2 représente sous forme d'histogramme le suivi du rendement en lactones produites, à savoir un mélange de (R)-5,6-dihydro-6-pentyl-2H-pyran-2-one et/ou de (4R,6R)-4-hydroxy-6-pentyl-tetrahydro-2H-pyran-2-one, après 3, 5 et 7 jours de culture, par chacune des souches d'*Aureobasidium* testées par fermentation à 25°C.

De même, la figure 3 représente sous forme d'histogramme le suivi du rendement en lactones produites, à savoir un mélange de (R)-5,6-dihydro-6-pentyl-2H-pyran-2-one et/ou de (4R,6R)-4-hydroxy-6-pentyl-tetrahydro-2H-pyran-2-one, après 3, 5 et 7 jours de culture, par chacune des souches d'*Aureobasidium* testées par fermentation à 30°C.

Ainsi, on évalue la production en lactones par chacune des souches testées par le procédé selon l'invention.

L'effet de l'augmentation de la température de fermentation lors de l'étape de culture du procédé selon l'invention a ainsi été testé pour toutes les souches en termes de rendement en lactones produites.

A cet effet, parmi toutes les souches testées, on remarque, tel qu'illustré par les figures 2 et 3 notamment, que la souche CBS 771.97 se révèle être la plus productrice en lactones dès J3, que ce soit à 25°C ou à 30°C, ainsi qu'à J5 et jusqu'à J7 également.

Par fermentation à 25°C, le rendement en lactones produites est au moins 3 fois supérieurs pour la souche CBS 771.97 par rapport aux autres souches à J3 et J5, et au moins 2 fois supérieurs à J7.

De même, la différence de rendement est encore plus marquante par fermentation à 30°C, le rendement en lactones produites est au moins 10 fois supérieurs pour la souche CBS 771.97 par rapport aux autres souches à J3, au moins 5 fois supérieurs à J5, et au moins 7 fois supérieurs à J7.

On observe également que certaines souches sont insensibles à la température en terme de production. C'est le cas par exemple de la souche CBS 117.466 qui ne produit de sucrolipide et donc de lactone à aucune des deux températures de fermentation testées ou encore la souche CBS 123.33, qui produit une quantité minime de lactones et semblable à 25°C et 30°C.

En outre, l'augmentation de la température de fermentation pour certaines souches semble avoir un effet négatif sur le rendement en lactones. On l'observe nettement pour la souche CBS 147.97 où l'augmentation de la température de fermentation de 25°C à 30°C présente un effet drastique en termes de production. En effet, la souche ne produit plus du tout de lactone à 30°C, comparé à 25°C où le rendement atteint 2,8 g/L à J7. D'autres souches, comme par exemple la souche CBS 584.75 sont également sensibles à l'augmentation de la température : elle produit 6 fois moins de lactones à 30°C par rapport à 25°C à J7. D'ailleurs, cet effet négatif est notable dès J3. La souche CBS 585.75 fait également partie du groupe de souches dont l'augmentation de la température présente un effet négatif sur le rendement total en lactones.

Toutefois, de manière surprenante et avantageuse, il apparait qu'un effet positif de l'augmentation de la température ne soit observé que pour la souche CBS 771.97, qui produit encore plus de lactones par fermentation à 30°C par rapport à 25°C avec une augmentation significative de 22,6% à J7, contrairement à toutes les souches criblées.

On observe donc une spécificité de la souche CBS 771.97 ou ses souches apparentées (clade N°7) selon le procédé selon l'invention.

### Exemple 2 :

Une étude complémentaire sur l'effet de l'augmentation de la température de fermentation dans le procédé selon l'invention sur le rendement en lactones a été plus particulièrement réalisée pour la souche plus particulièrement d'intérêt, à savoir *Aureobasidium pullulans* CBS 771.97, à partir des mêmes conditions opératoires que celles de l'étude comparative ci-avant.

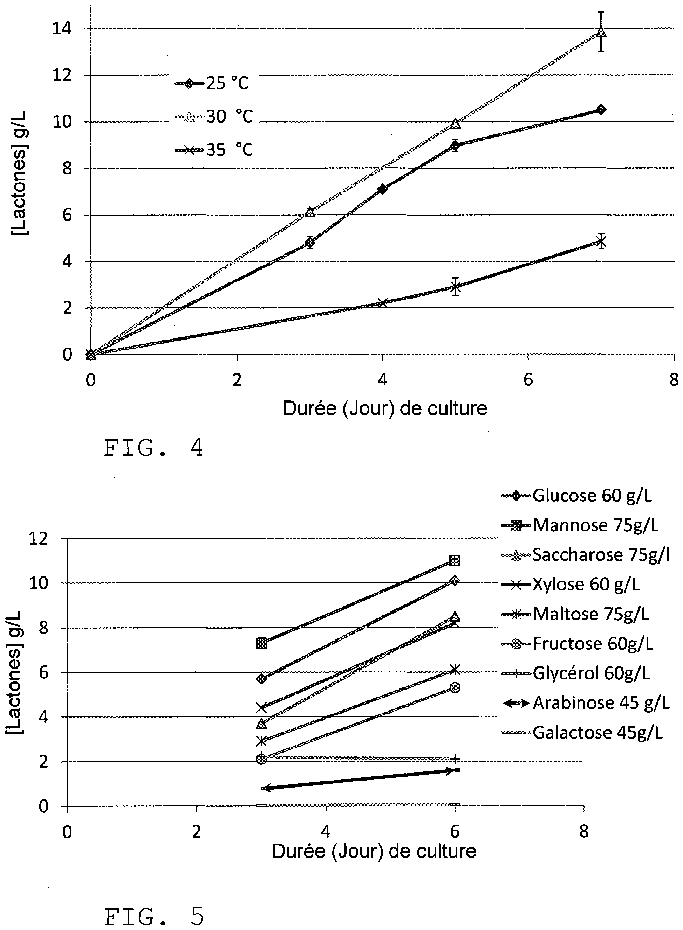

Les rendements en lactones produites obtenus dans cette étude complémentaire sont répertoriés dans le tableau 4 ci-dessous et illustrés par la figure 4 qui représente l'effet de la température de l'étape de culture sur la production de lactones selon un exemple du procédé selon l'invention pour la souche *Aureobasidium pullulans* CBS 771.97.

**Tableau 4 : Etude de l'augmentation de la température sur la production en lactones**

| **T(°C)** | **lactones totales (g/L)** | | | |
|---|---|---|---|---|
| | **J3** | **J4** | **J5** | **J7** |
| 25 | 4,8 | 7,1 | 9 | 10,5 |
| 30 | 6,1 | | 9,9 | 13,9 |
| 35 | | 2,2 | 2,9 | 4,9 |

D'après les résultats obtenus, on observe un effet positif de la température de fermentation à 30°C, par comparaison à 25°C et 35°C, sur le rendement en lactones produites à partir de la souche *Aureobasidium pullulans* CBS 771.97 dès J3, et plus particulièrement à J7 avec des augmentations significatives respectivement de 32,4% et 183,7%.

De plus, les rendements obtenus dans cette étude complémentaire mettent en évidence la bonne reproductibilité du procédé selon l'invention en ce que les rendements obtenus en lactones produites à 25°C et 30°C dans des conditions de culture semblables varient de seulement 8,3% (J3) et 1% (J7) à 25°C, et sont identiques (J3) et varient de seulement 6,9% (J7) à 30°C entre les résultats obtenus dans cette étude complémentaire et ceux obtenus dans l'étude comparative de l'exemple 1 pour cette souche CBS 771.97.

Les conditions opératoires mises en oeuvre par le Demandeur et la présence en particulier de calcium dans le milieu de production aqueux favorisent donc la production de sucrolipides et par suite de lactones, en particulier de (R)-(-)-massoia lactone, par la souche *Aureobasidium pullulans* CBS 771.97. Les conditions opératoires réalisées dans le procédé selon l'invention qui consistent en des opérations communément réalisées par l'homme du métier, sont ainsi très faciles à mettre en oeuvre.

Le procédé selon l'invention présente en outre une bonne reproductibilité et une productivité améliorée (quantité de lactone formée et vitesse de production améliorées) en lactones, en particulier en (R)-(-)-massoia lactone. A cet effet, on remarque qu'avec cette souche CBS 771.97 ou ses souches apparentées, les metabolites produits ne sont avantageusement pas rapidement dégradés après avoir atteint un maximum et peuvent être transformés en (R)-5,6-dihydro-6-pentyl-2H-pyran-2-one (ou (R)-(-)-massoia lactone) et/ou (4R,6R)-4-hydroxy-6-pentyl-tetrahydro-2H-pyran-2-one.

Le Demandeur a également mis en évidence l'effet de la source de carbone (exemple 3), tel qu'illustré dans la figure 5, sur la production de lactones selon un exemple du procédé selon l'invention à partir de la souche *Aureobasidium pullulans* CBS 771.97.

### Exemple 3 :

L'effet de la source de carbone sur la production de lactones a été testé sur la base des conditions opératoires de l'exemple 1 à partir de la souche *Aureobasidium pullulans* CBS 771.97 par culture à 25°C. Il est plus particulièrement illustré dans la figure 5.

On observe dans la figure 5 que, parmi les sources de carbone testées, l'arabinose et le galactose (à 45 g/L) ne permettent pas une production significative de lactones selon le procédé selon l'invention. Il est important de noter que les rendements obtenus sont également semblables voire inférieurs à des concentrations d'arabinose ou de galactose de 60 ou encore 75 g/L.

En revanche, la source de carbone du milieu de production du procédé selon l'invention choisie parmi le glycérol, le fructose, le maltose, le xylose, le saccharose, le glucose et le mannose, à une concentration de 60 ou 75 g/L, donnent des rendements significatifs en lactones produites d'au moins 2 g/L pour le glycérol après 6 jours de culture par fermentation à 25°C et jusqu'à 11 g/L pour le mannose à J6 également.

D'après les résultats obtenus, la source de carbone préférentiellement utilisée est le glucose à 60 g/L qui présente le meilleur rapport métabolites produits (lactones produites)/coût.

### Exemple 4 :

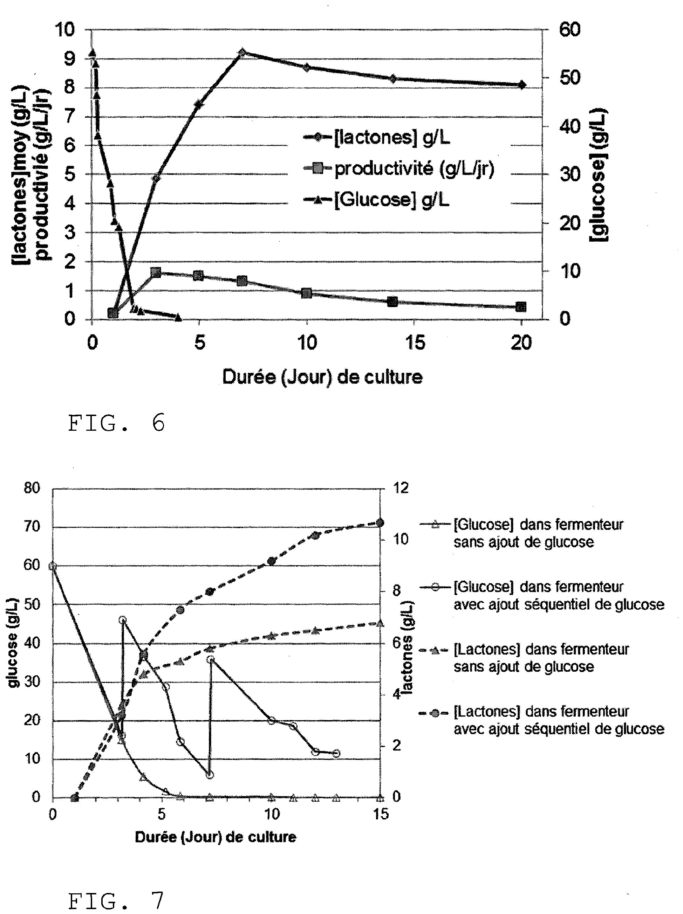

Le Demandeur a également réalisé le suivi de la production de lactones, de la productivité et de la consommation de glucose sur la base des conditions opératoires de l'exemple 1 à partir de la souche *Aureobasidium pullulans* CBS 771.97 par culture à 25°C sur une période de 20 jours de culture, tels qu'illustrés dans la figure 6.

Le maximum de production de lactones, de l'ordre de 9 g/L, a été atteint après 7 jours de culture. Compte tenu des erreurs de mesure, on peut considérer qu'il y a un plateau entre 7 et 10 jours.

En terme de productivité, le maximun est atteint à 5 jours puis chute.

La disparition du glucose dans le milieu de culture semble concomittante à la chute de la productivité.

### Exemple 5 :

A cet effet, selon un mode de réalisation avantageux du procédé selon l'invention, l'ajout séquentiel du milieu de production, et plus particulièrement de la source de carbone, et notamment de glucose, au cours de l'étape de culture a été testé à partir de la souche *Aureobasidium pullulans* CBS 771.97 sur la base des conditions opératoires de l'exemple 1 en introduisant une quatrième étape de préculture. La préculture réalisée à l'étape 3 sert à l'ensemencement de trois fioles qui servent à leur tour de préculture à deux fermenteurs de 1L. L'étape par culture est ensuite réalisée à 28°C en fermenteur, avec une aération de 0,5 vvm et une agitation de 500 rpm, sur une période de 15 jours de culture.

Dans le premier fermenteur, la source de carbone, plus particulièrement le glucose, provient du milieu de production (60 g/L). Dans le second fermenteur, en plus des 60 g initialement contenus dans le milieu de production, 30 g de glucose sous forme d'une solution aqueuse stérile de 500 g/L ont été respectivement ajoutés pendant l'étape de culture à J3 puis J7.

Le suivi de la production de lactones et de la consommation de glucose sont plus particulièrement illustrés dans la figure 7.

On observe ainsi une augmentation de la quantité de lactones produites au court du temps lorsque le glucose est ajouté séquentiellement, à la fois plus importante et plus rapide qu'en absence d'ajout de glucose au cours de l'étape de culture. Ainsi, à J7, on observe une augmentation de 38% de la quantité de lactones produites, l'augmentation étant de 57% à J12.

L'ajout séquentiel du milieu de production, et plus particulièrement de la source de carbone, et notamment par exemple de glucose, au cours de l'étape de culture permet ainsi le maintien d'une productivité élévée sur une durée plus longue c'est-à-dire, dans cet exemple, supérieure à 5 jours.

Bien entendu, l'invention ne se limite pas aux modes de réalisation et aux exemples présentés ci-dessus et l'homme du métier, grâce à des opérations de routine, pourra être amené à réaliser d'autres modes de réalisation non décrits explicitement, qui entrent dans le cadre large de l'invention.

## Revendications

1. Procédé de production de lactones à partir d'une souche *d'Aureobasidium pullulans,* **caractérisé en ce qu'**il comprend les étapes suivantes selon lesquelles :
- on réalise une préculture de la souche *Aureobasidium pullulans* CBS 771.97 (obtenue auprès du CBS-KNAW Fungal Biodiversity Centre, Uppsalalaan 8, 3584 CT Utrecht, Pays-Bas) ou d'une des souches *Aureobasidium pullulans* qui appartient au même clade que la souche CBS 771.97 ;
- à partir d'un inoculum issu de la préculture, on réalise une culture par fermentation à une température comprise entre 20°C et 40°C pendant une période d'au moins 3 jours de manière à produire des métabolites, dans un milieu de production aqueux stérilisé contenant :
- une source de carbone ;
- une source d'azote ;
- une solution de sels minéraux ; et
- une source de calcium, à une concentration comprise entre 2 et 100 mM ; et
- à l'issue de la période de fermentation, on transforme les métabolites produits en un mélange de lactones comprenant la (R)-5,6-dihydro-6-pentyl-2H-pyran-2-one et/ou la (4R,6R)-4-hydroxy-6-pentyl-tetrahydro-2 H-pyran-2-one.

2. Procédé selon la revendication 1, **caractérisé en ce qu'**il comprend en outre une étape selon laquelle on transforme la (4R,6R)-4-hydroxy-6-pentyl-tetrahydro-2H-pyran-2-one obtenue en (R)-5,6-dihydro-6-pentyl-2H-pyran-2-one par déshydratation.

3. Procédé selon la revendication 1 ou 2, **caractérisé en ce qu'**il comprend en outre une étape selon laquelle on isole la (R)-5,6-dihydro-6-pentyl-2H-pyran-2-one obtenue par extraction par un solvant organique suivie d'une distillation, ou par extraction au CO₂ supercritique, ou par hydrodistillation.

4. Procédé selon l'une des revendications précédentes, **caractérisé en ce que** l'étape de préculture est réalisée à partir d'un cryotube de la souche *Aureobasidium pullulans* CBS 771.97 ou ses souches apparentées dans un milieu solide et/ou liquide choisi parmi un milieu à base d'extrait de malt ou un milieu YNB (« Yeast Nitrogen Base »).

5. Procédé selon l'une des revendications précédentes, **caractérisé en ce qu'**on inocule un volume de préculture de la souche *Aureobasidium pullulans* CBS 771.97 ou ses souches apparentées dans le milieu de production relatif à une Densité Optique (DO) initiale du milieu de culture comprise entre 0,5 et 2.

6. Procédé selon la revendication 5, **caractérisé en ce que** le volume de l'inoculum est compris entre 3 et 10% du volume du milieu de culture.

7. Procédé selon l'une des revendications précédentes, **caractérisé en ce que** l'étape de culture par fermentation est réalisée à une température de 25°C à 35°C.

8. Procédé selon l'une des revendications précédentes, **caractérisé en ce que** l'étape de culture par fermentation est réalisée sous agitation pendant une période de 3 à 20 jours.

9. Procédé selon l'une des revendications précédentes, **caractérisé en ce que** la concentration en calcium dans le milieu de production est de 50 mM.

10. Procédé selon l'une des revendications précédentes, **caractérisé en ce que** la source de calcium est choisie parmi du carbonate de calcium CaCO₃ précipité pur à au moins 98% et comportant au moins un oligo-élément, solubilisé en condition acide et rajouté à la solution de sels minéraux extemporanément, ou du chlorure de calcium CaCl₂ en présence d'une solution d'oligo-élément(s).

11. Procédé selon la revendication 10, **caractérisé en ce que** l'oligo-élément est choisi parmi le manganèse, cuivre, cobalt, fer, zinc, strontium, plomb, mercure, chlore, bore, molybdène et soufre, pris seul ou en combinaison.

12. Procédé selon l'une des revendications précédentes, **caractérisé en ce que** :
- la source de carbone est choisie parmi le glucose, le mannose, le saccharose, le xylose, le maltose, le fructose et le glycérol, pris seul ou en combinaison ;
- la source d'azote est choisie parmi le nitrate d'ammonium NH₄NO₃, le nitrate de sodium NaNO₃, le sulfate d'ammonium (NH₄)₂SO₄, les peptones ou un acide aminé naturel, pris seul ou en combinaison, préférentiellement le nitrate d'ammonium NH₄NO₃ ;
- les sels minéraux en solution sont choisis parmi les ions magnésium, phosphate, potassium, sodium, chlorure, sulfate, pris seul ou en combinaison.

13. Procédé selon l'une des revendications précédentes, **caractérisé en ce que** le milieu de production contient en outre une source de vitamine.

## Patentansprüche

1. Verfahren zur Herstellung von Lactonen aus einem *Aureobasidium-pullulans*-Stamm, **dadurch gekennzeichnet, dass** es die folgenden Schritte umfasst, gemäß denen:
- eine Vorkultur des *Aureobasidium-pullulans*-Stamms CBS 771.97 (erhalten im CBS-KNAW Fungal Biodiversity Centre, Uppsalalaan 8, 3584 CT Utrecht, Niederlande) oder eines der *Aureobasidium-pullulans*-Stämme, der der gleichen Glade wie der Stamm CBS 771.97 angehört, durchgeführt wird;
- ausgehend von einem Inokulum, das aus der Vorkultur stammt, eine Kultur durch Fermentierung bei einer Temperatur im Bereich zwischen 20 °C und 40 °C während eines Zeitraums von mindestens 3 Tagen durchgeführt wird, um Metaboliten in einem sterilisierten wässrigen Herstellungsmedium herzustellen, enthaltend:
- eine Kohlenstoffquelle;
- eine Stickstoffquelle;
- eine Lösung aus Mineralsalzen; und
- eine Kalziumquelle mit einer Konzentration im Bereich zwischen 2 und 100 mM; und
- am Ende des Fermentierungszeitraums die hergestellten Metaboliten in eine Mischung aus Lactonen umgewandelt werden, umfassend das (R)-5,6-Dihydro-6-pentyl-2H-pyran-2-on und/oder das (4R,6R)-4-Hydroxy-6-pentyl-tetrahydro-2H-pyran-2-on.

2. Verfahren nach Anspruch 1, **dadurch gekennzeichnet, dass** es außerdem einen Schritt umfasst, gemäß dem das erhaltene (4R,6R)-4-Hydroxy-6-pentyl-tetrahydro-2H-pyran-2-on durch Dehydrierung in (R)-5,6-Dihydro-6-pentyl-2H-pyran-2-on umgewandelt wird.

3. Verfahren nach Anspruch 1 oder 2, **dadurch gekennzeichnet, dass** es außerdem einen Schritt umfasst, gemäß dem das erhaltene (R)-5,6-Dihydro-6-pentyl-2H-pyran-2-on durch Extraktion durch ein organisches Lösemittel, gefolgt von einer Destillierung, oder durch Extraktion durch superkritisches CO₂ oder durch Hydrodestillation isoliert wird.

4. Verfahren nach einem der vorhergehenden Ansprüche, **dadurch gekennzeichnet, dass** der Schritt des Vorkultivierens ausgehend von einem Kryoröhrchen des *Aureobasidium-pullulans*-Stamms CBS 771.97 oder seiner ähnlichen Stämme in einem festen und/oder flüssigen Medium, ausgewählt aus einem Medium auf der Grundlage von Malz oder einem YNB ("Yeast Nitrogen Base")-Medium durchgeführt wird.

5. Verfahren nach einem der vorhergehenden Ansprüche, **dadurch gekennzeichnet, dass** ein Volumen Vorkultur des *Aureobasidium-pullulans*-Stamms CBS 771.97 oder seiner ähnlichen Stämme im Herstellungsmedium mit Bezug auf eine anfängliche optische Dichte (DO) des Kulturmediums im Bereich zwischen 0,5 und 2 beimpft wird.

6. Verfahren nach Anspruch 5, **dadurch gekennzeichnet, dass** das Volumen des Inokulums im Bereich zwischen 3 und 10 % des Volumens des Kulturmediums liegt.

7. Verfahren nach einem der vorhergehenden Ansprüche, **dadurch gekennzeichnet, dass** der Schritt des Kultivierens durch Fermentierung bei einer Temperatur von 25 °C bis 35 °C durchgeführt wird.

8. Verfahren nach einem der vorhergehenden Ansprüche, **dadurch gekennzeichnet, dass** der Schritt des Kultivierens durch Fermentierung unter Rühren während eines Zeitraums von 3 bis 20 Tagen durchgeführt wird.

9. Verfahren nach einem der vorhergehenden Ansprüche, **dadurch gekennzeichnet, dass** die Kalziumkonzentration im Herstellungsmedium 50 mM beträgt.

10. Verfahren nach einem der vorhergehenden Ansprüche, **dadurch gekennzeichnet, dass** die Kalziumquelle ausgewählt ist aus ausgefälltem Calciumcarbonat CaCO₃ mit einer Reinheit von mindestens 98 % und umfassend mindestens ein Oligoelement, das in saurem Zustand solubilisiert und der Lösung aus Mineralsalzen unmittelbar zugegeben wird, oder Kalziumchlorid CaCl₂ in Anwesenheit einer Lösung von Oligoelementen.

11. Verfahren nach Anspruch 10, **dadurch gekennzeichnet, dass** das Oligoelement ausgewählt ist aus Mangan, Kupfer, Kobalt, Eisen, Zink, Strontium, Blei, Quecksilber, Chlor, Bor, Molybdän und Schwefel, allein oder in Kombination.

12. Verfahren nach einem der vorhergehenden Ansprüche, **dadurch gekennzeichnet, dass**:
- die Kohlenstoffquelle ausgewählt ist aus Glukose, Mannose, Saccharose, Xylose, Maltose, Fruktose und Glycerol, alleine oder in Kombination;
- die Stickstoffquelle ausgewählt ist aus Ammoniumnitrat NH₄NO₃, Natriumnitrat NaNO₃, Ammoniumsulfat (NH₄)₂SO₄, Peptonen oder einer natürlichen Aminosäure, allein oder in Kombination, vorzugsweise Ammoniumnitrat NH₄NO₃;
- die Mineralsalze in Lösung ausgewählt sind aus den Magnesium-, Phosphat-, Kalium-, Natrium-, Chlorid-, Sulfationen, allein oder in Kombination.

13. Verfahren nach einem der vorhergehenden Ansprüche, **dadurch gekennzeichnet, dass** das Herstellungsmedium außerdem eine Vitaminquelle enthält.

## Claims

1. Method for producing lactones from an *Aureobasidium pullulans* strain, **characterised in that** it comprises the following steps according to which:
- a preculture of the *Aureobasidium pullulans* strain CBS 771.97 is produced (obtained from the CBS-KNAW Fungal Biodiversity Centre, Uppsalalaan 8, 3584 CT Utrecht, Netherlands) or one of the *Aureobasidium pullulans* strains belonging to the same clade as the CBS 771.97 strain;
- from an inoculum issuing from the preculture, a culture is produced by fermentation at a temperature of between 20°C and 40°C for a period of at least 3 days so as to produce metabolites, in a sterilised aqueous production medium containing:
- a source of carbon;
- a source of nitrogen;
- a mineral salts solution; and
- a source of calcium, at a concentration of between 2 and 100 mM; and
- at the end of the fermentation period, the metabolites produced are transformed into a mixture of lactones comprising (R)5,6-dihydro-6-pentyl-2H-pyran-2-one and/or (4R,6R)-4-hydroxy-6-pentyl-tetrahydro-2H-pyran-2-one).

2. Method according to claim 1, **characterised in that** it further comprises a step according to which the (4R,6R)-4-hydroxy-6-pentyl-tetrahydro-2H-pyran-2-one) obtained is transformed into (R)5,6-dihydro-6-pentyl-2H-pyran-2-one by dehydration.

3. Method according to claim 1 or claim 2, **characterised in that** it further comprises a step according to which the (R)5,6-dihydro-6-pentyl-2H-pyran-2-one obtained is isolated by extraction by means of an organic solvent followed by distillation, or by extraction with supercritical CO₂, or by hydrodistillation.

4. Method according to any of the preceding claims, **characterised in that** the preculture step is carried out using a cryotube of the *Aureobasidium pullulans* strain CBS 771.97 or the related strains thereof in a solid and/or liquid medium chosen from a medium based on malt extract or a YNB (yeast nitrogen base) medium.

5. Method according to any of the preceding claims, **characterised in that** a volume of preculture of the *Aureobasidium pullulans* strain CBS 771.97 or the related strains thereof are inoculated in the production medium relating to an initial optical density (OD) of the culture medium of between 0.5 and 2.

6. Method according to claim 5, **characterised in that** the volume of the inoculum is between 3% and 10% of the volume of the culture medium.

7. Method according to any of the preceding claims, **characterised in that** the step of culture by fermentation is carried out at a temperature of 25°C to 35°C.

8. Method according to any of the preceding claims, **characterised in that** the step of culture by fermentation is carried out under stirring for a period of 3 to 20 days.

9. Method according to any of the preceding claims, **characterised in that** the calcium concentration in the production medium is 50 mM.

10. Method according to any of the preceding claims, **characterised in that** the calcium source is chosen from at least 98% pure precipitated calcium carbonate CaCO₃ comprising at least one trace element, solubilised in acid condition and added to the solution of mineral salts extemporaneously, or calcium chloride CaCl₂ in the presence of a solution of trace elements(s).

11. Method according to claim 10, **characterised in that** the trace element is chosen from manganese, copper, cobalt, iron, zinc, strontium, lead, mercury, chorine, boron, molybdenum and sulfur, taken alone or in combination.

12. Method according to any of the preceding claims, **characterised in that**:
- the carbon source is chosen from glucose, mannose, saccharose, xylose, maltose, fructose and glycerol, taken alone or in combination;
- the nitrogen source is chosen from ammonium nitrate NH₄NO₃, sodium nitrate NaNO₃, ammonium sulfate (NH₄)₂SO₄, peptone or a natural amino acid, taken alone or in combination, preferentially ammonium nitrate NH₄NO₃;
- the mineral salts in solution are chosen from magnesium, phosphate, potassium, sodium, chlorine and sulfate ions, taken alone or in combination.

13. Method according to any of the preceding claims, **characterised in that** the production medium also contains a vitamin source.
